# EUROPEAN PATENT APPLICATION

(11) **EP 3 162 424 A1**
(43) Date of publication of application: **03.05.2017**
(21) Application number: 16164315.0
(22) Date of filing: 07.04.2016
(51) Int. Cl.: B01D 29/01, B01D 69/10

(54) **FILTRATION MEMBRANE HOLDER**

(30) Priority: 23.10.2015 TW 104216990
(71) Applicant: Fiberpure Co., Ltd., Taipei City 114 (TW)
(72) Inventor: Lai, Wen-Yea, 114 Taipei City (TW)
(74) Representative: Lang, Christian

(57) **Abstract**

A filtration membrane holder includes a top plate and a bottom plate. The top plate includes crossbars of a first "*" shape and a matching part at the side of a periphery of the top plate. The bottom plate is a cavity structure and includes crossbars of a second "*" shape and a matching cavity at the side of a periphery of the bottom plate. When the top plate and the bottom plate are assembled, the matching part of the top plate is correspondingly combined with the matching cavity of the bottom plate to deposit the top plate within the cavity structure of the bottom plate and to make the crossbars of the first "*" shape and the ones of second "*" shape be assembled tightly. The area and strength of the filtration membrane holder may support a filtrate membrane and prevent it from curving and shedding, as well as pass blood plasma without resistance.

## Description

### FIELD OF THE INVENTION

The present invention relates to a filtration membrane holder, and more particularly relates to a filtration membrane holder that may fix a filtrate membrane onto two ends of an adsorber and prevent resin from exhausting along with blood plasma out of the adsorber.

### BACKGROUND OF THE INVENTION

Blood purification is a usual and effective treatment. Principle of blood purification is to exhaust blood out human body and treat blood with extracorporeal circulation. During extracorporeal circulation, target molecules within blood can be cleaned by passing a filtration membrane or resin for adsorption, and then cleaned blood after the extracorporeal circulation is sent back human body. Thus, blood purification treatment has applied onto people suffering from kidney, liver, or hyperglycemia diseases, as well as anti-rejection during organ transplantation surgeries.

There are two approaches for clinic blood purification: whole blood adsorption and plasmapheresis adsorption. Whole blood adsorption is simple and convenient but has drawback of useless adsorption because of high possibility on coagulation and too many interference factors. Plasmapheresis adsorption adopts separation and reflux process without blocking blood flow from coagulation and has no issues of blood coagulation, because only molecules are adsorbed and removed in plasma. Furthermore, plasmapheresis adsorption is of higher adsorption effect because it is not subject to blood cells and has good contact between adsorbed subjects and target molecules.

Most of adsorbers used in blood plasma separation and adsorption utilize resin to adsorb target molecules, but the resin within the adsorbers may be leaked out because of pressure by machines of adsorbers and then enter into human body. So manufacturing of adsorber is very important. Furthermore, approaches for preventing the resin from leaking usually generate much flow resistance and makes adsorbers fail to filtrate. Thus, a filtrate membrane is usually used to intercept resin and enables resin stay within adsorber. However, too small flow rate and delaying treatment are issues to be resolved.

### SUMMARY OF THE INVENTION

Accordingly, a filtration membrane holder is provided for the one used in an adsorber. The filtration membrane holder may fix a filtrate membrane onto two ends of the adsorber and prevent resin from leaking along with blood plasma out of the adsorber.

Accordingly, a filtration membrane holder with structures of "*" shape that may be against mechanical pressure without deformation or bending and does not form extra resistance on blood plasma filtration.

The " * " shape is a star shape with at least three bars crossing in a central region of the star shape wherein each end of a bar being spaced apart from the neighboring bars by 60° around an axes through the central region at the intersection of the crossing bars.

Accordingly, the area and the strength of the filtration membrane holder may support a filtrate membrane and prevent it from curving and shedding, as well as pass blood plasma without resistance.

Accordingly, a filtration membrane holder includes a top plate and a bottom plate. The top plate includes crossbars of a first " * " shape and a matching part at the side of a periphery of the top plate. The bottom plate is a cavity structure and includes crossbars of a second " * " shape and a matching cavity at the side of a periphery of the bottom plate. When the top plate and the bottom plate are assembled, the matching part of the top plate is correspondingly combined with the matching cavity of the bottom plate to deposit the top plate within the cavity structure of the bottom plate and to make the crossbars of the first " * " shape and the ones of second " * " shape be assembled tightly.

### BRIEF DESCRIPTION OF THE DRAWINGS

Fig. 1 is a schematic diagram illustrating the components of a filtration membrane holder according to the present invention.
Fig. 2 is a schematic diagram illustrating the front view, the side view, and the back view of the top plate according to the present invention.
Fig. 3 is a schematic diagram illustrating the front view, the side view, and the back view of the bottom plate according to the present invention.

### DETAILED DESCRIPTION OF THE PREFERRED EMBODIMENT

The present invention discloses a filtration membrane holder, and its parts made by mechanical process are well known by one having general knowledge in the field. Thus, there will not be illustrated in detailed in the following paragraphs. The presently described embodiments will be understood by reference to the drawings, but the sizes or ratios of components shown in drawings are not intended to limit the scope of the disclosure.

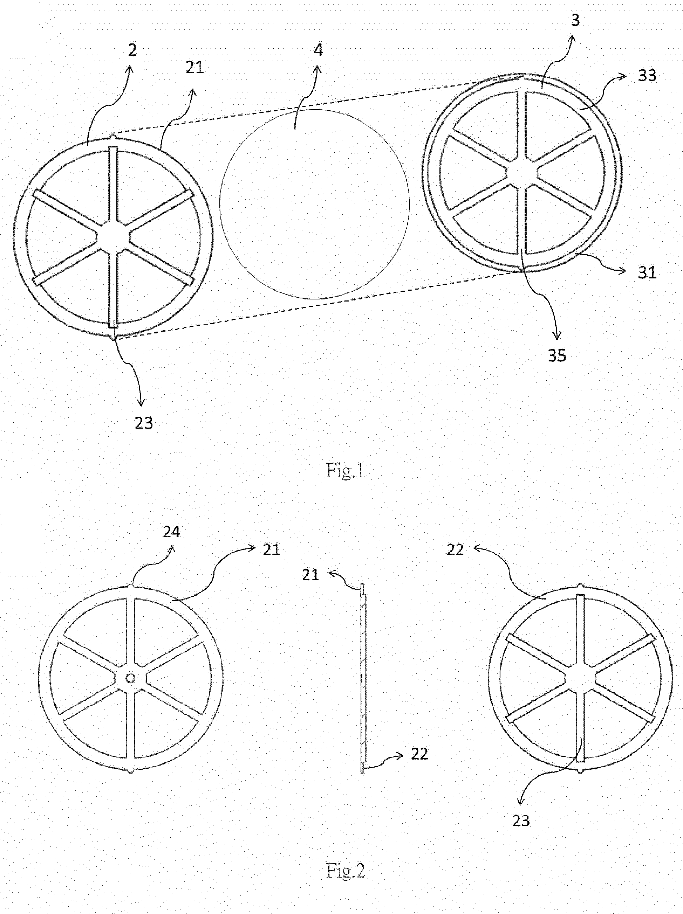

First, Fig. 1 is a schematic diagram illustrating the components of a filtration membrane holder according to the present invention. In Fig. 1, a filtration membrane holder 1 includes a top plate 2 and a bottom plate 3 combined with each other that are configured to fix a filtration membrane 4.

Fig. 2 is a schematic diagram illustrating the front view, the side view, and the back view of the top plate according to the present invention. In Fig. 2, there are crossbars of a first " * " shape 23 within the periphery of the top plate 2, and other parts except the first " * " shape 23 are hollow within the periphery of the top plate 2. Next, the top plate 2 includes a top surface 21 and a bottom surface 22 corresponding with each other. Furthermore, there is a matching part 24 at the side of the periphery of the top plate 2, and the matching part 24 may be deposited at one end of the any crossbar of the first " * " shape 23.

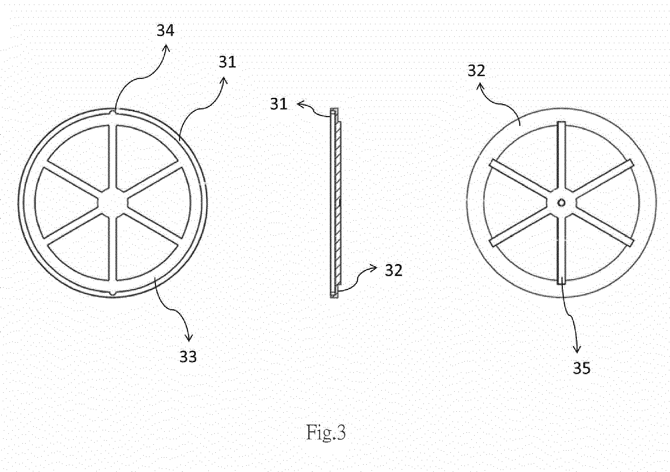

Fig. 3 is a schematic diagram illustrating the front view, the side view, and the back view of the bottom plate according to the present invention. In Fig. 3, the bottom plate 3 includes a top surface 31 and a bottom surface 32 corresponding with each other. The bottom plate 3 includes a cavity structure 33 of the top surface 31 and crossbars of a second " * " shape 35.

Other parts except the second " * " shape 35 are hollow within the periphery of the bottom plate 3. Next, there is a matching cavity 34 at the side of the periphery of the bottom plate 3 to be shapely corresponding to the matching part 24 of the top plate 2. The matching cavity 34 may be deposited at one end of the any crossbar of the first " * " shape 35. It is noted that the top plate 2 may have the matching parts 24 in pairs that are deposited at the ends of the crossbars at opposite sides, as well as the matching cavities 34 in pairs. That is, the matching parts 24 in pairs are spaced 180 degrees away from each other on the top plate 2, as well as the matching cavities 34 in pairs of the bottom plate 3. Besides, the matching part 24 may be a projecting piece and the matching cavity 34 may be a notch piece. Alternatively, the matching part 24 may be a notch piece and the matching cavity 34 may be a projecting piece.

Please refer to Fig. 1 again, when the top plate 2, the filtration membrane 4, and the bottom plate 3 are assembled together, the filtration membrane 4 may be putted on the top surface 21 of the top plate 2 or the top surface 31 of the bottom plate 3, and the bottom surface 22 of the top plate 2 faces and is deposited onto the cavity structure 33 of the top surface 31 of the bottom plate 3, so as to deposit the filtration membrane 4 between the top plate 2 and the bottom plate 3. Next, the matching parts 24 of the top plate 2 are correspondingly combined with the matching cavity 34 of the bottom plate 3 to make the crossbars of the first " * " shape 23 and the ones of second " * " shape 35 overlap with each another to be assembled tightly. The filtration membrane 4 is fixed between the top plate 2 and the bottom plate 3 and exposed to area outside the overlapping area of the first " * " shape 23 and the second " * " shape 35, so as to filtrate blood plasma when blood plasma passes through the filtration membrane 4. Accordingly, by the first " * " shape 23 and the second " * " shape 35, the filtration membrane 4 is fixed and loaded even surface tension. As a result, an issue that the velocity of blood plasma and impact pressure of fluid result in curving and shedding of a traditional filtration membrane may be resolved by the designs of the present invention.

Accordingly, the filtration membrane holder 1 of the present invention fixes the filtration membrane 4 onto two ends of a housing of an adsorber by depositing the filtration membrane 4 between the top plate 2 and the bottom plate 3, which efficiently prevents resin within the adsorber from leaking along with blood plasma.

While the invention has been described in terms of what is presently considered to be the most practical and preferred embodiments, it is to be understood that the invention needs not be limited to the disclosed embodiments. On the contrary, it is intended to cover various modifications and similar arrangements included within the spirit and scope of the appended claims which are to be accorded with the broadest interpretation so as to encompass all such modifications and similar structures.

## Claims

1. A filtration membrane holder comprising:
a top plate comprising crossbars of a first " * " shape and at least a matching part at the side of a periphery of the top plate; and
a bottom plate being a cavity structure and comprising crossbars of a second " * " shape and at least a matching cavity at the side of a periphery of the bottom plate; when the top plate and the bottom plate are assembled, the matching part of the top plate is correspondingly combined with the matching cavity of the bottom plate to deposit the top plate within the cavity structure of the bottom plate and to make the crossbars of the first " * " shape and the ones of second " * " shape be assembled tightly.

2. The filtration membrane holder of claim 1, further comprising a filtration membrane deposited between the top plate and the bottom plate.

3. The filtration membrane holder of claim 1, wherein the top plate and the bottom plate have circular shapes.

4. The filtration membrane holder of claim 1, wherein area of the top plate outside the crossbars of the first " * " shape is hollow.

5. The filtration membrane holder of claim 1, wherein area of the bottom plate outside the crossbars of the second " * " shape is hollow.

6. The filtration membrane holder of claim 1, wherein the matching part is a projecting piece and the matching cavity is a notch piece.

7. The filtration membrane holder of claim 1, wherein the matching part is a notch piece and the matching cavity is a projecting piece.
